# EUROPEAN PATENT APPLICATION

(11) **EP 1 852 088 A1**
(43) Date of publication of application: **07.11.2007**
(21) Application number: 06009369.7
(22) Date of filing: 05.05.2006
(51) Int. Cl.: A61F 2/06, B29C 63/34

(54) **Shape memory devices**

(71) Applicant: Mnemoscience GmbH, 52531 Uebach-Palenberg (DE)
(72) Inventor: Lendlein, Andreas, 14167 Berlin (DE)
(74) Representative: Grünecker, Kinkeldey, Stockmair & Schwanhäusser Anwaltssozietät

(57) **Abstract**

The present invention concerns shape memory devices, methods of producing same and the use of these materials.

## Description

The present invention concerns shape memory devices, methods of producing same and the use of these materials.

### Prior art

Shape memory polymers are an interesting class of materials, which have received considerable attention in recent years. Shape memory functionality is the ability of a material to temporarily fix a second shape after an elastic deformation and only recover its original shape after application of an external stimulus. While this effect is one-way, reversible shape changes induced by cooling and heating, i.e. a two-way effect may also be realized.

The advantageous and intriguing properties of these materials are in particular the possibility to initiate a desired change in shape by an appropriate external stimulus, so that an original shape, after deformation, is re-established, and the possibility to deform and program these materials so that highly specific configurations and shape changes can be obtained. The deformed shape is often called the temporary shape in the art. The phenomenon is a functionality and not an inherent material property. The effect/functionality results from a combination of polymer structure and specific functionalization processes.

The first materials known to provide this functionality were metallic shape memory alloys. In the recent past, shape memory polymers have been developed. Typical shape memory polymers are, for example, phase segregated linear block copolymers, having a hard segment and a switching (soft) segment. Important representatives of these types of materials are disclosed in the international publications WO 99/42147 and WO 99/42528. These materials employ as external stimulus for initiating the recovery of the original shape a change in temperature, usually a temperature rise. Shape memory polymers being susceptible to other external stimuli are also known, such as the photosensitive shape memory polymers disclosed in WO 2004/062706.

For some applications it would however be desirable to have a material which displays a shape memory effect i n response to the exertion of mechanical forces (tensile stress, compression load etc), a stimulus towards which conventional shape memory polymers do not react by showing a shape memory effect (rather conventional shape memory polymers would show initially no response and finally, with strong mechanical forces, would show mechanical failure).

Accordingly the present invention aims at providing a material or composition or device which enables the selective initiation of a shape memory effect in response to the external stimulus "mechanical force".

### Brief description of the invention

The present invention solves the above object with the shape memory device as defined in claim 1. Preferred embodiments are outlined in claims 2 to 7. Furthermore the present invention provides the method for preparing such shape memory devices as outlined in claim 8 as well as the use as defined in claims 9 and 10.

### Description of the figures

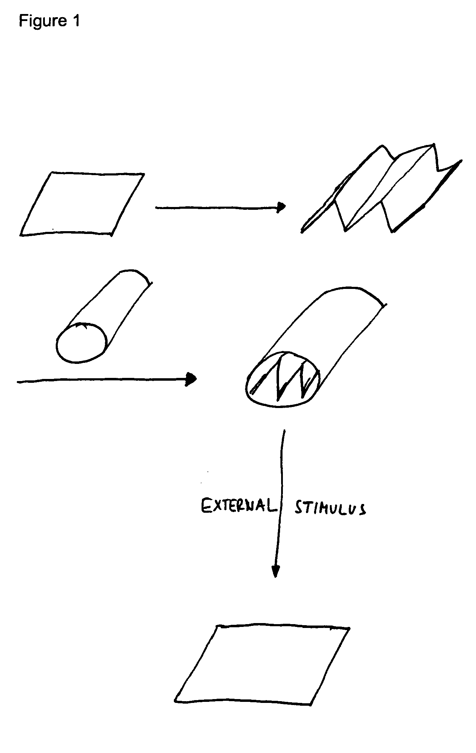

Figure 1 shows the use of a first material in sheet form which is then folded or rolled into a deformed shape. This deformed shape is the fixed by a second material, shown in figure 1 in the shape of a tube. After application of the suitable external stimulus the first material recovers the original non deformed shape.

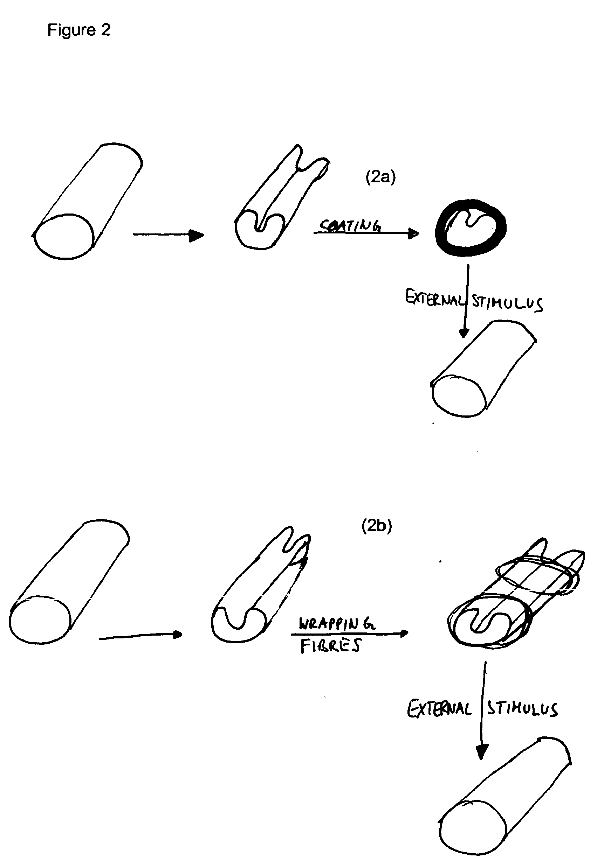

Figure 2 shows the use of the first material in the form of a tube, such as a stent or the like. This tube is then deformed by compressing and the deformed shape is fixed by a coating of the second material (Figure 2a) or the use of fibers of the second material, which are wrapped around the article formed from the first material (Figure 2b). After application of the suitable external stimulus the first material recovers the original non deformed shape.

### Detailed description of the present invention

As defined in claim 1, the shape memory devices of the present invention are sensitive towards an external stimulus, in particular mechanical forces, i.e. they show a shape memory effect after having been subjected to mechanical forces. As is further derivable from claim 1 the device comprises a first material which can memorize at least one shape, i.e. in accordance with the usual designation in the art the permanent shape. This material, in the device, is present in the deformed, i.e. temporary shape. However, in accordance with the present invention it is not required that the deformed state, i.e. the temporary shape (the designation as used again corresponds to the usual designation employed in the art for shape memory polymers) is fixed by means of interactions within the material (for example by chemical or physical interactions of soft or switching segments of shape memory polymers) since the device in accordance with the present invention comprises an additional second material fixing the deformed shape. In this respect the present invention in particular requires that the first material is elastic, in order to allow a recovery of the permanent shape after release of the fixation provided by the second material.

Preferred first materials are accordingly elastomeric polymer networks, having a low glass transition temperature and being highly flexible. Further examples of preferred first materials are shape memory polymers with a switching temperature below the temperature at which the shape memory device in accordance with the present invention is to be used.

The device in accordance with the present invention accordingly fixes the deformed shape by an additional second material which may for example be provided in the form of a coating, partially or completely covering the article made from the first material. This second material displays a sufficient mechanical strength and physical integrity so that the temporary shape is secured. However, the second material is selected so that the application of a suitable external stimulus leads to a decrease of the mechanical strength of the second material or to the removal, partially or completely of the second material, so that the deformed shape cannot be maintained anymore. Instead the first material, no longer fixed by the second material, recovers its initial permanent shape, i.e. the deformed shape is lost and the permanent shape is formed.

As indicated above a suitable external stimulus in particular is a mechanical manipulation, such as a compression or a tensile stress. A further alternative is the application of a solvent in which selectively only the second material may be dissolved or at least swollen, so that the mechanical fixation is removed. Such a swelling leads to the formation of a gel phase of the second material so the mechanical integrity required for fixing the first material in the deformed shape is no longer given. The second material may for example be selected from brittle materials which show a sufficient integrity and/or cohesion that the deformed shape can be fixed, even against the internal forces within the first material. When this second material however is subjected to a mechanical manipulation as mentioned above, the integrity and/or cohesion of the second material is disturbed so that the second material can no longer hold the first material in the deformed shape. In accordance with the present invention it is however not only envisaged to use second materials which are sensitive towards a compression or tensile load, i.e. mechanical forces, but also which loose, as outlined above, the required integrity and/or cohesion upon application of other stimuli, such as the solvent sensitive materials mentioned above, examples of which are polyethylene glycol and polyvinyl alcohol.

Examples of suitable second materials are materials which soften due to heating, so that the above described effect occurs after having subjected the device to a heat treatment. A further option are pH sensitive materials, i.e. materials which even disintegrate after having been subjected to a suitable change in pH value (in particular for devices which are used in liquid containing environments). Other examples are light sensitive second materials or materials which are susceptible towards a hydrolytic degradation or an enzymatic degradation. In accordance with the present invention it is only necessary that the second material is able to fix and secure the deformed shape and that the second material is susceptible towards an external stimulus so that the first material, after the second material has been subjected to such a stimulus, recovers the remembered, i.e. permanent shape. Typical representatives of such suitable materials for the second material to be employed in accordance with the present invention are thermoplastic polymers.

The first material to be employed in accordance with the present invention may be any material which is able to maintain at least one shape in memory, i.e. which is able to recover the original shape after a deformation (and the fixation of the deformed shape by means of the second material). Suitable examples thereof are shape memory polymers as for example illustrated in the prior art references mentioned above. However, as outlined above, any material which is able to remember one shape may be employed in accordance with the present invention. Accordingly the present invention also contemplates to use a s first material, as a Iready indicated above, elastic materials, in particular rubber materials of natural or synthetic origin. Also such elastic materials, such as natural or synthetic rubber, including EPDM materials and the like, are materials which, after an elastic deformation, display the ability to return to the non-deformed state after the external force (i.e. in the present invention the restraining coating of the second material) fixing the deformed shape is removed. Preferred in this respect are rubber materials in the form of polymer networks having main chain segments providing a domain having a rather low glass transition temperature. At the same time it is preferred when these materials display only a minimal hysterises.

One further possibility in this respect is the use of a shape memory polymer which has been programmed and accordingly provides one permanent shape and at least one temporary shape (depending on the number of switching segments) so that in addition to the permanent shape and the temporary shape as enabled by the shape memory polymer a further deformed shape is made possible. In this embodiment the temporary shape as programmed onto the shape memory polymer is deformed further and this additional deformed shape is then fixed in accordance with the present invention using the second material. Accordingly in such an embodiment the first shape change occurs when the second material is no longer able to secure the deformed shape - the shape memory polymer returns to the temporary shape from which the permanent shape may be recovered upon initiating the shape memory effect of the shape memory polymer.

However, the present invention mainly is concerned with the fixation of a deformed state of a first material, which is able to memorize the original shape, using the second material, so that the device is able to recover this original shape when the second material no longer secures the deformed shape.

As indicated above, the second material partially or completely covers or at least fixes the article formed from the first material in the deformed shape. The type of coating, such as coating pattern, coating thickness etc. depends from the desired end use and the type of the first materials as well as the type of article and the degree of deformation. Complete coatings might in particular be required when the first material displays a strong tendency to recovering the original shape, while partial coatings may be suitable in particular in fields of application where the second material is rather expensive so that only the minimum required amount is to be used. However, these illustrative explanations shall not be construed as limitation since the skilled person will be in a position to determine the appropriate type of coating for the desired use and selected composition. A further alternative is the use of fibers or bands of the second material as well as sheets thereof which are used to be wrapped around the article formed from the first material being in the deformed shape. Thereby the deformed shape may be fixed as well without providing a coating of the second material onto the article formed from the first material.

As outlined above the second material may be selected from suitable materials providing a desired sensitivity towards an external stimulus. The following options are in particular envisaged by the present invention:
1.) thermo-sensitive materials application of heat softens the second material so that first material returns to permanent shape, i.e. non-deformed shape
2.) light-sensitive materials application of flight s oftens or degrades the second material so that first material returns to permanent shape, i.e. non-deformed shape
3.) solvent-sensitive materials application of solvent selectively softens or removes the second material so that first material returns to permanent shape, i.e. non-deformed shape
4.) pH-sensitive materials variation of pH-value softens, degrades or removes the second material so that first material returns to permanent shape, i.e. non-deformed shape
5.) materials sensitive towards a magnetic field application of magnetic field softens the second material so that first material returns to permanent shape, i.e. non-deformed shape

The second material furthermore may be selected to have suitable properties, such as biocompatible materials, erodable materials, materials which degrade, for example by hydrolytic or enzymatic processes, crystalline, semicrystalline or amorphous materials and the like, depending in particular from the desired end use of the device formed.

The shape memory device in accordance with the present invention may be prepared in a conventional manner using the forming techniques described in the art for shape memory polymers. The first material has to be provided in the original i.e. permanent shape for the desired article of manufacture. Subsequently the article is deformed until the desired deformed shape is obtained, which in turn then is fixed u sing the second material, for example by applying a partial or complete coating using conventional techniques. The article obtained accordingly is fixed in the deformed shape and the original shape can only be recovered by applying an external stimulus as indicated above.

The shape memory devices in accordance with the present invention may in particular be used in applications where a change in shape in response to an external mechanical force is suitable, for example in pressure sensors. Other applications are areas where a shape change in response to a tensile force or compression force is desired, for example sensors, but also medical devices as well as other articles of manufacture, such as toys etc. Further fields of application are medical devices, such as in particular stents, which may be fixed in a compressed, i.e. small diameter shape, by the second material, which in turn then is softened or removed after insertion so that the stent recovers its original shape

## Claims

1. Shape memory device, comprising a first material, able to memorize an original shape and being present in a deformed shape, and a second material, fixing the first material in the deformed shape, wherein the second material looses its ability to fix the first material in the deformed shape upon application of an external stimulus.

2. Shape memory device in accordance with claim 1, wherein the external stimulus is a mechanical force, selected among tensile forces, and compression forces.

3. Shape memory device in accordance with claim 1 or 2, wherein the second material partially or completely covers the first material.

4. Shape memory device in accordance with claim 1, 2, or 3, wherein the first material is a shape memory polymer.

5. Shape memory device in accordance with claim 1, 2, or 3, wherein the first material is a rubber material.

6. Shape memory device in accordance with any of claim 1 or 3 to 5, wherein the external stimulus is a temperature increase, or light irradiation, including UV irradiation.

7. Shape memory device in accordance with any of claim 1 or 3 to 5, wherein the external stimulus is a treatment with liquids, including water, acidic or basic solutions, and enzyme containing solutions.

8. Shape memory device in accordance with any of claims 1 to 7, wherein the second material is selected among thermoplastic polymers.

9. Shape memory device in accordance with any of claims 1 to 8, wherein the device is selected among sensors and medical devices, in particular stents.

10. Use of a shape memory device in accordance with any of claims 1 to 9 in a sensor, or in a medical device.
